# EUROPEAN PATENT APPLICATION

(11) **EP 4 800 122 A1**
(43) Date of publication of application: **02.09.2026**
(21) Application number: 25161234.7
(22) Date of filing: 03.03.2025
(51) Int. Cl.: C12Q 1/6806, C12Q 1/6827, C12Q 1/6883

(54) **METHOD OF ANALYZING A SAMPLE OF BIOLOGICAL MATERIAL USING A PIPETTING STATION TO DETECT MOSAIC GENETIC VARIANTS WITH VERY LOW FREQUENCY OF OCCURRENCE**

(30) Priority: 28.02.2025 PL 45134525
(71) Applicant: Gdanski Uniwersytet Medyczny, 80-210 Gdansk (PL); Fundació Institut d'Investigació en Ciències de la Salut Germans Trias i Pujol, 08916 Badalona, Barcelona (ES)
(72) Inventor: Piotrowski, Arkadiusz, 83-031 Rózyny (PL); Koczkowska, Magdalena, 80-126 Gdansk (PL); Horbacz, Monika, 80-280 Gda sk (PL); Filipowicz, Natalia, 80-262 Gdansk (PL); Wojdak, Agata, 80-126 Gdansk (PL); Prokopiuk, Justyna, 21-523 Dabrowica Duza (PL); Castellanos Perez, Elisabeth, 08027 Barcelona (ES)
(74) Representative: JD&P Patent Attorneys Joanna Dargiewicz & Partners

(57) **Abstract**

The subject of the invention is a method for analyzing a biological material sample using a pipetting station to detect mosaic genetic variants at a very low frequency in genes. The method involves the isolation of DNA from a peripheral blood sample, followed by the preparation of DNA libraries for duplex sequencing and subsequent bioinformatic analyses. The method is characterized in that: a) DNA samples are prepared and mechanical DNA fragmentation is performed using an ultrasonic device; b) The resulting DNA fragments of approximately 500 base pairs undergo enzymatic reactions, adapter ligation, incubation, and purification using magnetic beads, where the enzymatic reactions include repairing the ends of fragmented DNA, ligation of the dedicated adapters, and amplification using PCR; c) Amplification and hybridization of dedicated molecular probes targeting the human genome region are carried out; d) The quality and quantity of the obtained DNA libraries are verified, followed by sequencing. The results are analyzed using bioinformatic tools, producing a list of genetic variants along with their frequency in the studied gene(s). Steps b) and c) of the method are performed using the Bravo NGS pipetting station from Agilent Technologies.

## Description

The presented invention relates to method for analyzing a sample of biological material using the Agilent Bravo NGS pipetting station to detect mosaic genetic variants at very low frequencies in genes associated with the pathogenesis of selected neurocutaneous disorders.

In genetics, mosaicism refers to the presence of two or more populations of cells with distinct genotypes within a single individual. Depending on the type of cells harboring the genetic variant, mosaicism can be classified as follows: i) somatic mosaicism - when the genetic variant is present exclusively in somatic cells; ii) germline mosaicism - when the genetic variant is present exclusively in germ cells; iii) gonosomal mosaicism - when the genetic variant arises at an early stage of embryogenesis and is present in both somatic and germ cells.

With the advances of high-resolution molecular techniques, mosaicism has been shown to play a key role in numerous genetic diseases, influencing their onset, progression, and inheritance. One group of genetic disorders in which mosaicism is frequently observed includes cancer-predisposing syndromes, such as neurocutaneous disorders, historically referred to as phakomatoses. Phakomatoses comprise a heterogeneous group of over 70 distinct diseases characterized by skin lesions and/or tumor formation in various organs, particularly the nervous system. Individuals with these conditions have an increased risk of developing cancer compared to the general population, necessitating close surveillance, including regular imaging examinations, to enable early detection and personalized clinical management.

Among the phakomatoses, schwannomatoses can be distinguished based on their associated constitutional pathogenic variants, including NF2-related schwannomatosis (caused by pathogenic variants in the *NF2* gene) and *SMARCB1-* or LZTRI-related schwannomatosis (caused by pathogenic variants in the *SMARCB1* or *LZTR1* genes, respectively). Patients with schwannomatoses develop benign tumors derived from Schwann cells, which typically arise from nerves responsible for hearing and balance. These tumors often lead to progressive hearing loss, tinnitus, balance disturbances, and various other neurological symptoms. Due to the overlapping clinical presentation of different types of schwannomatoses, genetic testing is required for accurate diagnosis. Precise molecular classification is crucial, as the type of schwannomatosis determines the genetic counseling and clinical management strategies offered to patients. Additionally, ongoing clinical trials for targeted therapies focus on specific molecular pathways, further underscoring the need for an accurate genetic diagnosis to guide personalized treatment approaches.

Due to the high prevalence of mosaicism (approximately 25% of patients with suspected NF2-related schwannomatosis present with a mosaic form of the disease), routine molecular diagnostics using available methods remains challenging. The current approach to the differential diagnosis of schwannomatoses involves analyzing DNA obtained from both blood samples and at least two tumors originating from two anatomically distinct lesions. Targeted sequencing of the *NF2* gene in DNA isolated from peripheral blood lymphocytes, based on the results of genetic analyses of corresponding tumor samples - provided that a pathogenic *NF2* variant has been identified in the tumor - can aid in establishing a precise diagnosis. However, obtaining tumor material for molecular testing might be difficult due to its location within the nervous system, which complicates surgical removal or biopsy, and consequently, hinders accurate diagnosis. Therefore, improving the sensitivity of methods for detecting mosaic genetic variants in easily accessible material, such as DNA isolated from peripheral blood, is of critical importance.

A similarly high rate of somatic mosaicism (approximately 15%) has been also observed in patients with tuberous sclerosis caused by pathogenic loss-of-function variants in the *TSC1* or *TSC2* genes. Tuberous sclerosis is characterized by an increased predisposition to the development of tumors, primarily hamartomas, that can affect multiple systems. It is also associated with skin manifestations, seizures, delayed psychomotor development, and other neurological complications. Patients with mosaic pathogenic variants in *TSC1*/*TSC2* typically exhibit a milder clinical presentation, which complicates diagnosis based solely on clinical criteria - particularly in sporadic cases without a family history of the disease. Given the high risk of malignancy in these individuals, early diagnosis is crucial to ensure appropriate clinical surveillance and management, along with the implementation of targeted treatments when possible.

Standard next-generation sequencing (NGS), commonly used in routine molecular diagnostics, is limited by sequencing error rates, making it inadequate for identifying very low-frequency mosaic genetic variants, particularly when tumor material is unavailable for analysis. Duplex sequencing, a modification of NGS, offers much higher accuracy by utilizing information from both DNA strands, allowing for the detection of very low-frequency variants directly in blood-derived DNA. This eliminates the need for genetic testing of DNA isolated from tumor samples.

Duplex sequencing was introduced into laboratory practice more than a decade ago (Schmitt et al. Detection of Ultra-Rare Mutations by Next-Generation Sequencing. Proc Natl Acad Sci U S A. 2012;109(36):14508-13. PMID: 22853953), however its popularity has only increased significantly in recent years. This technology employs a double barcoding strategy to independently label both strands of the original DNA molecule, allowing each strand to be tracked individually during library preparation and sequencing. Based on these labels, reads are grouped into families, and a variant is considered as a true variant only if appears in reads from both strands of the original DNA - in contrast to standard NGS methods that rely on information from only one strand. Consequently, duplex sequencing effectively eliminates background noise, achieving error rates as low as 10⁻⁶ to 10⁻⁷. Although the complexity of protocol has limited its routine clinical application, automation now simplifies and significantly reduces the library preparation process, making the method more suitable for widespread use in both clinical and research settings.

Laboratory automation uses technologies to streamline laboratory processes that are traditionally performed manually. Preparing libraries for NGS sequencing, including duplex sequencing, is a multi-step procedure that requires high precision and consistency from the operator. The quantity and quality of the generated library are critical to the success of subsequent sequencing steps. For this reason, the process has been more often carried out using laboratory pipetting robots. These large-scale devices are ideal for routine and time-consuming NGS library preparation protocols. Several solutions such as Tecan, Eppendorf, Hamilton, Beckman-Coulter, and Agilent are available on the market, all designed to automate labor-intensive laboratory processes. Some companies offering pipetting robots also introduce ready-to-use protocols for the automation of library preparation processes using dedicated reagent kits available in their commercial offer. Currently, there are no protocols specifically designed for the automation of duplex sequencing library preparation processes available on the market. Such a solution has not been also reported in the literature.

The manual preparation of duplex libraries is demanding, time-consuming, and is characterized by significant standard deviations of results due to the lack of repeatability of pipetting, even by skilled personnel, which is a technical challenge. Therefore, there is a need for automation of this process to address these issues by improving pipetting precision, reducing library preparation time, and increasing throughput by processing more samples simultaneously.

The aim of the invention was to provide a highly-sensitive method for analyzing a biological material sample, serving as a potential diagnostic tool for detecting low-frequency mosaicism, as demonstrated in the differential diagnosis of phakomatoses.

The subject of the invention is a method for analyzing a sample of biological material using a pipetting station to detect mosaic genetic variants at a very low-level frequency in genes. The method involves isolating DNA from a peripheral blood sample, preparing DNA libraries for duplex sequencing, and performing further bioinformatic processing, characterized by the following:
a) DNA samples are prepared, and subjected to mechanical DNA fragmentation using an ultrasonic device.
b) The obtained DNA fragments, approximately 500 base pairs in length, undergo enzymatic reactions, adapter ligation, incubation, and purification using magnetic beads.

The enzymatic reactions include repair of the fragmented DNA ends, ligation of dedicated adapters, and amplification through PCR.
c) Dedicated molecular probes targeting the human genome region are amplified and hybridized.
d) The quality and quantity of the obtained DNA libraries are checked, and then sequencing is performed. The raw data is analyzed using bioinformatics tools, ultimately producing a list of genetic variants along with their frequency in the studied gene(s), while b) and c) are performed using an automated computational device: the Bravo NGS pipetting station from Agilent Technologies.

Preferably, in the method according to the invention, the hybridization step is carried out using molecular probes that cover coding regions, adjacent non-coding sequences within 50 nucleotides on both sides of the exons, and regions of selected pathogenic variants located deep in intronic sequences. These sequences are selected from the *TSC1, TSC2, NF2, LZTR1,* and *SMARCB1* genes, wherein the *TSC1* and *TSC2* genes are associated with the molecular pathogenesis of tuberous sclerosis, and the *NF2, LZTR1,* and *SMARCBI* genes are associated with the molecular pathogenesis of schwannomatosis.

Preferably, the method according to the invention detects mosaic variants at a low-level, in the range of 1 to 5%, and at a very low-level of <1%, in DNA isolated from peripheral blood leukocytes of patients suspected of having selected neurocutaneous diseases.

The advantage of the method according to the invention is the detection of genetic variants at the low (1-5%) and very low (<1%) mosaicism levels, while ensuring high reproducibility of results. The method increases throughput and reduces the time required for library preparation by 64% compared to standard manual sample processing. It also potentially reduces the initial DNA input while still ensuring the minimum amount of DNA required for PCR reaction. Analysis of the standard deviations of DNA concentrations at different stages of the library preparation process demonstrated that automation reduces pipetting errors by 2.2-fold compared to the manual method. As a result, not only is the reproducibility of results improved, but also the consumption of reagents is reduced, which lowers the costs of the analysis.

The method according to the invention also reduces the risk of contamination, which is particularly important in the case of highly sensitive methods, such as duplex sequencing. The experiments confirmed that the developed method meets diagnostic requirements in terms of sensitivity, while maintaining quality equal to or higher than manual methods. The final validation of the method was achieved through detailed tests with real samples, which is a key step in the process of implementing automation. The developed protocols have been validated on selected genes associated with the molecular pathogenesis of specific neurocutaneous disorders, such as *NF2, SMARCB1, LZTR1, TSC1,* and *TSC2.* However, they can also be applied in the molecular analysis of other genes, not related to phakomatoses, using duplex sequencing technology. To enhance diagnostic sensitivity, molecular probes have been designed to cover not only the coding regions of genes and adjacent non-coding intronic sequences within 50 nucleotides flanking exons, but also regions of selected pathogenic variants located deep within intronic sequences (i.e., deep intronic variants) with documented pathogenic significance. Internal validation allowed us to determine the lower detection threshold for mosaic genetic variants at the level of 0.02%-0.09%, depending on the analyzed gene. Variants occurring below this threshold are considered reliable provided that at least two duplex consensus are formed. Such values ensure high diagnostic sensitivity and enable detection of an extremely low-level of mosaicism.

During the validation of the method, sequencing results of DNA isolated from both tumors and peripheral blood leukocytes of patients suspected of having the mosaic form of selected neurocutaneous diseases were compared. DNA sequencing from peripheral blood leukocytes revealed the presence of the same pathogenic variants that were previously detected in the tumor, but at a much lower level, which was undetectable in standard NGS analyses. The method according to the invention therefore enables the detection of pathogenic variants at a very low-level in an easily accessible material, such as blood. This is of particular importance in the diagnosis of patients with phakomatoses, as tumors in this group of diseases often occur in the central or peripheral nervous system, and due to their difficult anatomical location, collecting a tumor sample for molecular testing can be challenging or even impossible. In the case of patients with the mosaic form of the disease, searching for pathogenic variants in DNA isolated from peripheral blood using traditional diagnostic methods may lead to false negative results due to insufficient sensitivity of the analyses. By utilizing highly sensitive duplex sequencing, it is possible to obtain precise results from peripheral blood DNA analysis, providing reliable diagnoses for patients with the mosaic form of the disease and enabling the implementation of appropriate clinical treatment.

The subject of the invention is illustrated by the accompanying figures, in which:
Fig. 1 presents an example of the DNA sample dilution scheme (sample P913) used in the method according to the invention.
Fig. 2 presents a diagram illustrating the time (in minutes) required to complete each enzymatic step, recorded for 8 samples, where the diamond symbol represents the time for the manual method and the square symbol represents the method according to the invention.
Fig. 3 presents a diagram illustrating the time (in minutes) required for one operator to perform the hybridization process (Target Capture 1 [TC1] and Target Capture 2 [TC2]) while preparing 16 libraries for duplex sequencing, where the diamond symbol represents the time for the manual method and the square symbol represents the method according to the invention.
Fig. 4 presents a comparison of the results obtained during the hybridization step using the method according to the invention and the manual method, considering the concentrations obtained for individual samples after the PCR2 (X-axis) and PCR3 (Y-axis) stages.
Fig. 5 presents the correlation between the expected frequency of the alternative allele and the actual detected frequency of the alternative allele using the analysis method according to the invention. In all presented cases, a strong correlation between the expected and actual values is observed.

The invention is illustrated by the following embodiments.

### Example 1

### Comparison of the analysis of a sample processed using the method according to the invention and the manual procedure

In the embodiment of the method according to the invention, variant B of the Bravo NGS platform (Bravo Automated Liquid Handling Platform, Agilent Technologies) integrated with VWorks software enabling full control over the station was used. The Bravo NGS platform is equipped with a precise pipetting system, allowing for liquid dispensing within a volume range of 300 nL to 250 µL. This ensures high repeatability of results and minimizes the risk of laboratory errors. Variant B of the Bravo NGS platform is additionally equipped with modules, including the BenchCel 4R Microplate Handler, which enables automatic management of consumables, and the MiniHub, used for storing accessories and reagents during operations. The gene panels used in the studies were designed using xGENTM Custom Hybridization Capture Panels (5'-biotinylated, 2xTiling, probe length - 120 nucleotides; reference genome hg38) from Integrated DNA Technologies (IDT).

The general method of analyzing a sample of biological material using a pipetting station according to the invention comprises the following steps:
1) Preparation of a DNA sample and mechanical fragmentation of the DNA using an ultrasonicator, which generates DNA fragments of approximately 500 base pairs in length.
2) Enzymatic processes and DNA purification, which involve a series of enzymatic reactions and incubation steps performed on the Bravo NGS station. Each of these steps is followed by DNA purification using magnetic beads. The processes include the following steps: i) Repairing the ends of the fragmented DNA, ii) Ligation of the dedicated adapters, iii) Amplification by PCR.
3) Hybridization of molecular probes (target capture), which enables the analysis of selected regions of the human genome (for example, genes associated with phakomatoses, such as *NF2, SMARCB1, LZTR1, TSC1,* and *TSC2*). This process consists of the following steps: i) Vacuum concentration of a sample with a total amount of 1500 ng after amplification, followed by mixing with pre-designed blocking oligonucleotides, and then dissolving in hybridization buffer using the Bravo NGS station. ii) Hybridization in a thermal cycler, after which the samples are washed with buffers in the presence of streptavidin beads, amplified, and purified using magnetic beads. iii) A second hybridization process, which is repeated to increase the quantity and specificity of the product.
4) Quality control and quantitative analysis - at the end of the process, the quality and quantity assessment is performed for each sample, including analyzing the size of the DNA fragments and the concentration of the libraries. Subsequently, sequencing of the obtained DNA libraries is performed, followed by further bioinformatic analysis of raw data to generate a list of variants and their frequency in the studied gene(s).

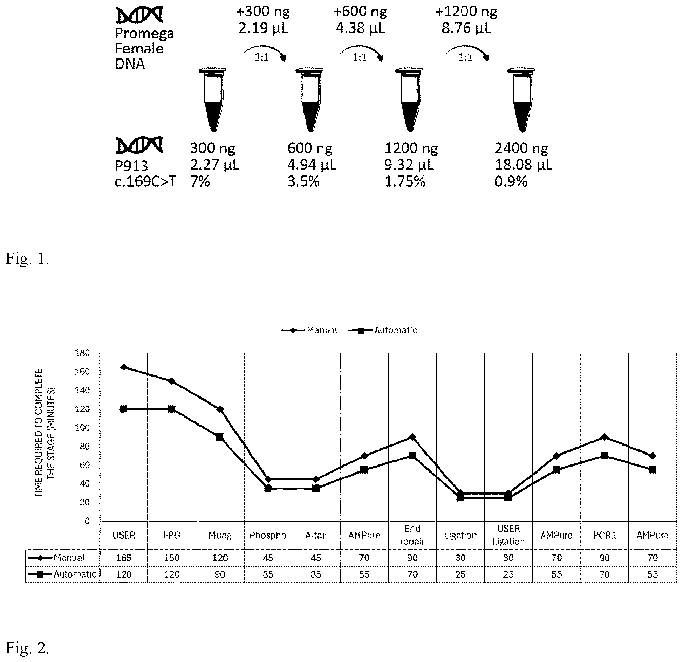

In the validation of the invention, we used DNA samples collected from patients for whom specific genetic variants had been previously identified, enabling the evaluation of the accuracy and effectiveness of the method according to the invention in conditions similar to real diagnostic applications. The experiment started with appropriate dilution of DNA samples, which were prepared based on results obtained using a less sensitive technique (standard NGS or direct Sanger sequencing), currently used as a diagnostic standard in clinical laboratories. This approach allowed for the reproduction of theoretical allele frequency ranges from 1.75% to 0.04% (Fig. 1). The tests were performed using three hybridization panels covering five genes: panel i) *NF2,* panel ii) *TSC1* and *TSC2,* and panel iii) *LZTR1* and *SMARCB1,* which enabled the assessment of the sensitivity of the method according to the invention for genes located in different *loci* of the genome.

The tests were performed on a DNA sample (sample P913) containing a specific pathogenic variant in the *NF2* gene, with an initial allele frequency of 7%. Sample P913 was diluted according to the scheme presented in Fig. 1, resulting in a final theoretical allele frequency of 0.9%. The method utilized commercially available, purified DNA from female (Human Genomic DNA: Female, catalog number G1521, Promega), with an initial concentration of 2000 ng in both samples. In the manual procedure, all operations, such as pipetting and reagent mixing, were performed by an experienced laboratory diagnostician. For the method according to the invention, the individual steps were carried out by a pipetting station under the supervision of a laboratory diagnostician. The automation process encompassed all stages of sample preparation, from pipetting and reagent mixing to incubation processes.

During the tests, full compliance with the experimental conditions for both approaches was ensured. Temperatures, process times, the number of PCR cycles, as well as incubation and hybridization parameters were carefully standardized to guarantee the comparability of the results. Special attention was given to the accuracy of pipetted liquid volumes and the uniformity of reagent mixing. After preparation, both samples were sequenced under identical conditions.

Scheme 1 presents all the steps involved in the duplex sequencing library preparation process
according to the invention, along with the list of developed protocols.
[A] ENZYMATIC STEPS
max. 1500 ng DNA after fragmentation
1_USER_Treatment.pro
2_FPG_Treatment.pro
3_Mung_Bean.pro
4_Phosphorylation.pro
5_A_tailing.pro
6_AMPure_Purification.pro
7_End_repair.pro
8_Adapter_Ligation.pro
9_Ligation_USER.pro
10_AMPure_Purification_after_Ligation.pro
11_PCR1.pro
12_AMPure_Purification_after_PCR1.pro
HYBRIDIZATION
max. 1500 ng DNA after PCR1
[B] OPTION UP TO 8 SAMPLES
13_TC1_TC2_Hybridization.pro
14_TC1_TC2_Streptavidin_Beads_Wash.pro
15_PostCapture_PCR2.pro
15_PostCapturePCR3.pro
16_AMPure_Purification_after_PCR2_PCR3.pro
[C] OPTION UP TO 16 SAMPLES
17_TC1_TC2_Hybridization.pro
18_TC1_TC2_Streptavidin_Beads_Wash.pro
19_PostCapture_PCR2.pro
19_PostCapturePCR3.pro
20_AMPure_Purification_after_PCR2_PCR3.pro

Schemes 1 and 2 present the designed protocol for automating the duplex sequencing library preparation process, along with the time-temperature profile. Protocols 1-12 cover the enzymatic steps, with the appropriate protocol selected via the form and interface in Panel A. Panels B and C depict the hybridization steps, offering two options: processing up to 8 samples (Panel B) or up to 16 samples simultaneously (Panel C).

Scheme 2 presents the individual steps of the method according to the invention, along with
the time-temperature profile.

ENZYMATIC STEPS

1 User Treatment
131μL DNA after Covaris
1h incubation in 37°C
Purification with AMPureXP

2 FPG Treatment
30μL DNA after User
1h incubation in 37°C
Purification with AMPureXP

3 MungBean Treatment
20μL DNA after FPG
30min incubation in 30°C
Purification with AMPureXP

4 Phosphorylation
20μL DNA after MungBean
30min incubation in37°C
(lid temp 47°C)
Purification with AMPureXP

5 A-tailing
30μL DNA after Phospho
30min incubation in 37°C
(lid temp 47°C)
Purification with AMPureXP

6 End-repair
50μL DNA after A-tailingu
(maximum1000 ng)
30min incubation in 20°C
30min incubation in 65°C
(lid temp >75°C)

7 Adapter Ligaiton
DNA after End-repair
15min incubation in 20°C
(lid temp off)

8 Ligation User
DNA after Ligation
15min incubation in 37°C
(lid temp 47°C)
Purification with AMPureXP

9 PCR1
240 ng DNA after User
95°C 4 min
98°C 20 s
65°C 20 s x8
72°C 45 s
72°C 5 min
(lid temp > 75°C)
next 2μL
NEBNext Universal (100 μM)
95°C 4 min
98°C 20 s
65°C 20 s x2
72°C 45 s
72°C 5 min
(lid temp > 75°C)
Purification with AMPureXP

HYBRIDIZATION ( TC 1 AND 2 )
Maximum 1500 ng after PCR1/ 20μL after TC1
1) Vacuum concentration with Cot-I-DNA, mws60 i mws61 ( blocking oligonucleotides)
2) Adding hybridization mix with probes, incubation for 10 minutes
3) Hybridization - 95°C for 30 s, next 65°C for 4h30 min (lid temp 100°C)
4) In the meantime - preparation of buffers for washing steps and its inkubation minimum
1h in 65°C
5) Streptavidine beads preparation and adding them to samples after hybridization is
completed
6) Incubation in 65°C for 45minutes (lid temp 100°C), mixing every 10-15 minutes
7) Washing with special buffers in 65°C on the first three streps, then washing at room
temperature
8) Posthybridization amplification PCR2 / PCR3 in following conditions:
95°C 4 min
98°C 20 s
65°C 20 s x10
72°C 30 s
72°C 3 min (lid temp > 75°C)
9) Purification with magnetic AMPureXP beads

Based on the final concentrations obtained during the enzymatic steps in both methods, the percentage error was calculated relative to the initial concentration of 2000 ng. Table 1 lists the concentration values for the manual procedure and method according to the invention, taking into account the results obtained at each individual step. The percentage error was then calculated to indicate the degree of deviation of the measured concentrations from the initial value. The percentage error was calculated using the following formula: $Percentage Error = \frac{\left|Measured Concentration-Initial Concentration\right|}{Initial Concentration} \times 100$

**Table 1. Summary of concentration values for the manual procedure and method according to the invention, taking into account the results obtained at each enzymatic step.**

| **Sample P913: dilution 0.9% variant c.169 C>T** | USER (ng) | Loss 1 (%) | FPG (ng) | Loss 2 (%) | Mung (ng) | Loss 3 (%) | A-tail (ng) | Loss 4 (%) | Ligation (ng) | Loss 5 (%) |
|---|---|---|---|---|---|---|---|---|---|---|
| **Manual** | 1545 | 23 | 1130 | 44 | 920 | 54 | 795 | 60 | 623 | 69 |
| **Automation** | 1740 | 13 | 852 | 52 | 625 | 69 | 523 | 74 | 500 | 75 |

The calculated percentage error reflects the differences between the initial value and the concentrations obtained after enzymatic steps. The results of these calculations allow for the assessment of the reproducibility of the method according to the invention in relation to the initial value. The data demonstrates that the efficiency of both methods, when calculated by means of material losses, is comparable. However, the automatic procedure is characterized by smaller percentage errors compared to the manual method. This is particularly evident in the first stage, where the percentage error for the manual method is 23%, while for the automatic method it is only 13%. Despite the significant decrease in concentration in both cases, smaller discrepancies are observed in the automatic method, especially after the stage marked as "Mung." The subsequent stages show a minimal decrease in concentration, significantly lower than in the case of the manual procedure. This indicates greater consistency and stability in the automatic method.

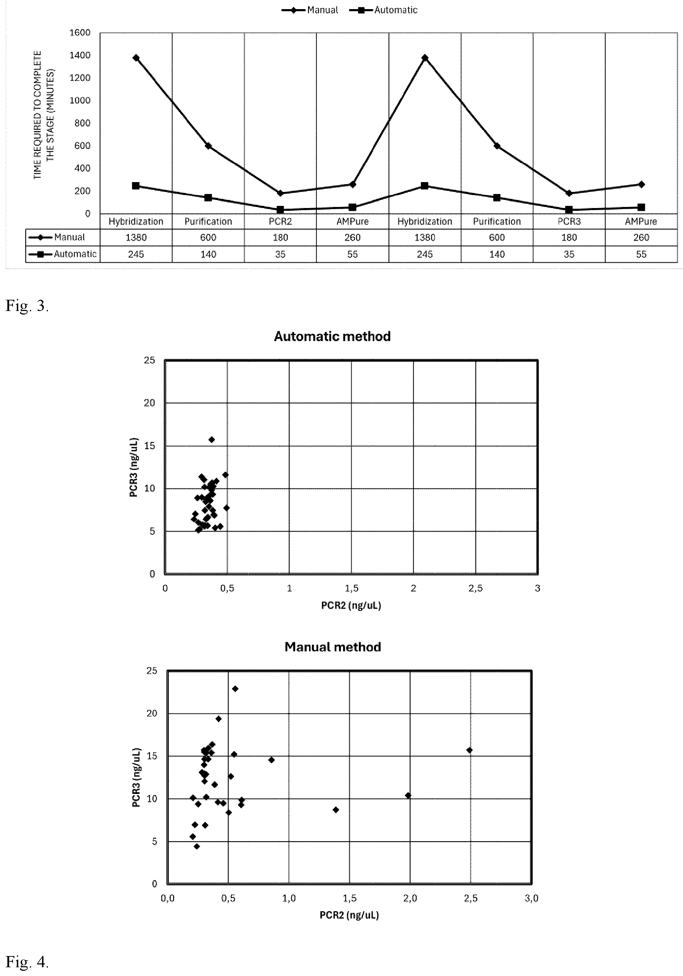

In addition, when analyzing a larger number of samples, automation proved to be more effective, enabling savings in both time and resources (Fig. 2 and Fig. 3). In the automated procedure, the detected frequency of the alternative allele matched the expected value (0.9%), whereas the manual method showed a 10% deviation from the expected value (0.77%).

**Table 2. Comparison of sequencing results for samples prepared using the manual method and the method according to the invention.**

| **Sample P913: dilution 0.9% variant c.169 C>T** | Expected frequency of the alternative allele | Detected frequency of the alternative allele | Depth of coverage for the alternative allele | Total sequencing depth at this position |
|---|---|---|---|---|
| **Manual** | 0.90% | 0.77% | 36 | 4686 |
| **Automation** | 0.90% | 0.90% | 63 | 7001 |

### Example 2

### Duplex sequencing method according to the invention using a hybridization panel targeting the NF2 gene locus

In subsequent experiments, the lowest frequencies of alternative alleles that could be detected by the procedure of the invention using a panel for the *NF2* gene were assessed. To achieve this, serial dilutions of the variant detected by another method (standard NGS or Sanger sequencing) in the patient sample were performed, prepared according to the procedure outlined in Fig. 1. The initial total amount of DNA in each sample was 2000 ng.

Table 3 shows the losses of genetic material at all enzymatic stages. The duplex sequencing method is generally characterized by high material losses, regardless of whether the procedure was carried out manually or automatically. As in the Example 1, the lowest losses were observed at the first enzymatic stage, while after the stage marked as "Mung," the losses were clearly smaller. The final amount of DNA obtained significantly exceeded the amount required for the first amplification (PCR1), which was 240 ng. Importantly, small differences in material losses between samples with different theoretical initial frequencies of the alternative allele indicate the repeatability of the protocols used.

**Table 3. Summary of concentration values for the method according to the invention using a hybridization panel targeting the NF2 gene locus.**

| **Sample ID** | USER (ng) | Loss 1 (%) | FPG (ng) | Loss 2 (%) | Mung (ng) | Loss 3 (%) | A-tail (ng) | Loss 4 (%) | Ligation (ng) | Loss 5 (%) |
|---|---|---|---|---|---|---|---|---|---|---|
| P274 1% | 1935 | 3 | 1240 | 38 | 815 | 59 | 695 | 60 | 478 | 76 |
| P274 0.5% | 1890 | 6 | 972 | 51 | 672 | 66 | 686 | 66 | 538 | 73 |
| P274_025% | 1815 | 9 | 1060 | 47 | 618 | 69 | 606 | 70 | 490 | 76 |
| P274 0.12% | 1740 | 13 | 1170 | 42 | 675 | 66 | 554 | 72 | 480 | 76 |
| P274 0.06% | 1890 | 6 | 1190 | 41 | 717 | 64 | 498 | 75 | 565 | 72 |
| P1539 0.7% | 1725 | 14 | 887 | 56 | 694 | 65 | 536 | 73 | 555 | 72 |
| P1539 0.35% | 1875 | 6 | 933 | 53 | 1050 | 48 | 401 | 80 | 503 | 75 |
| P1539 0.175% | 1935 | 3 | 1370 | 32 | 1110 | 45 | 477 | 76 | 633 | 68 |
| P1539 0.09% | 1860 | 7 | 1130 | 44 | 695 | 65 | 456 | 77 | 540 | 73 |
| P1539 0.04% | 1695 | 15 | 935 | 53 | 764 | 62 | 689 | 66 | 660 | 67 |
| **Mean** | **1836** | **8** | **1088** | **46** | **781** | **61** | **560** | **72** | **544** | **73** |
| **Median** | **1860** | **7** | **1088** | **46** | **717** | **64** | **554** | **72** | **540** | **73** |
| **Standard deviation** | **87.81** | **4.39** | **157.62** | **7.66** | **166.93** | **8.06** | **105.57** | **6.08** | **62.4** | **3.22** |

The results of the subsequent analyses, including amplification and hybridization steps, are presented in Table 4. These data indicate that the amount of genetic material after the PCR steps depends on the properties of the specific sample. After the first PCR (PCR1), the values obtained for samples from the P274 series were clearly higher compared to the P1539 series. The final amounts of genetic material after the third PCR (PCR3) differ between samples, but in both series, they were within comparable ranges, which confirms the effectiveness of the applied automated procedure.

**Table 4. Comparison of amplification and hybridization efficiency for samples from two series with different concentrations processed with the automated procedure according to the invention, using a hybridization panel targeting the NF2 gene locus.**

| **Sample ID** | After PCR1 (ng) | After PCR3 (ng) | Fragment length (bp) | Final library concentration (nM) |
|---|---|---|---|---|
| P274_1% | 4320 | 93 | 713 | 10 |
| P274_0.5% | 3600 | 120 | 693 | 13 |
| P274_0.25% | 4660 | 111 | 666 | 13 |
| P274_0.12% | 4480 | 123 | 676 | 14 |
| P274_0.06% | 4340 | 103 | 691 | 11 |
| P1539_0.7% | 3000 | 70 | 692 | 8 |
| P1539_0.35% | 2460 | 66 | 661 | 8 |
| P1539_0.175% | 2740 | 76 | 665 | 9 |
| P1539_0.09% | 2380 | 84 | 653 | 10 |
| P1539_0.04% | 3940 | 96 | 670 | 11 |

Tables 5 and 6 demonstrate the sequencing results, including both the expected and detected frequencies of the alternative allele for the samples used in the study. In both cases, small deviations between the expected and detected values were observed, which may be attributed to the lower sensitivity of the molecular methods initially used to determine the presence of the variant in the patient. However, the decrease in allele frequency follows the dilution scheme applied in both cases (Tables 5 and 6). These results validate the effectiveness of the automated method in detecting variants with frequencies below 1%, enabling the identification of alternative alleles at frequencies as low as 0.02%.

However, it is important to highlight that lower frequency values are associated with greater uncertainty, primarily due to the limited depth of coverage of the alternative allele. The data suggest that a safe cut-off threshold for analytical reliability is a frequency of 0.03%, or a minimum depth of coverage of 2 for the alternative allele. These parameters are critical for ensuring precise detection when using the tested *NF2* panel. Based on the results, it can be concluded that the automated procedure is highly sensitive, as evidenced by the predictability of material losses at each stage of the analysis and the consistent quality of the final samples, irrespective of variations in the initial frequency of the alternative allele.

**Table 5. Sequencing results of sample P274 prepared at five different dilution levels using the automation method according to the invention.**

| Expected frequency of the alternative allele P274 *NF2* - variant c.592C>T | Detected frequency of the alternative allele | Depth of coverage for the alternative allele | Total sequencing depth at this position |
|---|---|---|---|
| 1% | 0,55% | 32 | 5851 |
| 0.5% | 0.11% | 6 | 5568 |
| 0.25% | 0.09% | 6 | 6790 |
| 0.12% | 0.03% | 2 | 6129 |
| 0.06% | 0.03% | 2 | 6710 |

**Table 6. Sequencing results of sample P1539 prepared at five different dilution levels using the automation method according to the invention.**

| Expected frequency of the alternative allele P1539 *NF2* - variant c.169C>T | Detected frequency of the alternative allele | Depth of coverage for the alternative allele | Total sequencing depth at this position |
|---|---|---|---|
| 0.7% | 0.27% | 11 | 4101 |
| 0.35% | 0.25% | 9 | 3630 |
| 0.175% | 0.15% | 6 | 4008 |
| 0.09% | 0.07% | 3 | 4115 |
| 0.04% | 0.02% | 1 | 6127 |

### Example 3

### Duplex sequencing method according to the invention using a hybridization panel targeting the SMARCB1/LZTR1 genes loci

Similarly to Example 2, subsequent analyses assessed the lowest frequencies of alternative alleles that can be detected using the procedure according to the invention with a panel for two genes, *SMARCB1* and *LZTR1,* associated with the molecular pathogenesis of schwannomatosis. The procedure was carried out as described in Example 2, maintaining a similar initial concentration of the material (1600 ng). Table 7 presents the results obtained at the individual enzymatic stages, while data for the subsequent stages, including amplification and hybridization, are presented in Table 8. The sequencing results presented in Tables 9 and 10 demonstrate minor differences between the expected and detected frequencies of alternative alleles, consistent with the findings in Example 2. Nevertheless, the reduction in the allele frequency proceeded according to the assumed dilution scheme, and the obtained data confirm the effectiveness of the method in detecting variants with a frequency below 1.56%, with a detection limit for the *SMARCB1*/*LZTR1* genes of 0.05%.

In summary, the automation of the duplex sequencing library preparation process according to the invention ensures high consistency of results. The use of this procedure is especially beneficial in analyses that demand high precision, such as detecting variants with extremely low allele frequencies. Automation not only enhances reproducibility but also reduces human error, making it a reliable tool for detecting variants with frequencies below traditional detection limits. This method is especially valuable in clinical applications where accurate and sensitive detection is critical.

**Table 7. Summary of concentration values for the method according to the invention using a hybridization panel targeting the SMARCBI and LZTR1 gene loci.**

| **Sample ID** | USER (ng) | Loss 1 (%) | FPG (ng) | Loss 2 (%) | Mung (ng) | Loss 3 (%) | A-tail (ng) | Loss 4 (%) | Ligatio n (ng) | Loss 5 (%) |
|---|---|---|---|---|---|---|---|---|---|---|
| P131T 1.56% | 1452 | 12 | 791 | 52 | 599 | 64 | 470 | 72 | 395 | 76 |
| P131T 0.39% | 1280 | 22 | 722 | 56 | 584 | 65 | 368 | 78 | 323 | 80 |
| P131T 0.1% | 1289 | 19 | 691 | 57 | 541 | 66 | 323 | 80 | 275 | 83 |
| P174T 1.56% | 1461 | 18 | 922 | 48 | 547 | 69 | 447 | 75 | 328 | 82 |
| P174T 0.39% | 1515 | 9 | 768 | 54 | 756 | 55 | 447 | 73 | 353 | 79 |
| P174T 0.1% | 1440 | 28 | 716 | 64 | 486 | 76 | 459 | 77 | 283 | 86 |
| **Mean** | **1406** | **12** | **768** | **52** | **586** | **63** | **419** | **74** | **326** | **80** |
| **Median** | **1446** | **10** | **745** | **53** | **566** | **65** | **447** | **72** | **326** | **80** |
| **Standard deviation** | **97.72** | **6.84** | **83.66** | **5.38** | **92.32** | **6.85** | **59.31** | **3.06** | **44.62** | **3.46** |

**Table 8. Comparison of amplification and hybridization efficiency for samples from two series with different concentrations processed with the automated procedure according to the invention, using a hybridization panel targeting the SMARCBI and LZTR1 gene loci.**

| **Sample ID** | After PCR1 (ng) | After PCR3 (ng) | Fragment length (bp) | Final library concentration (nM) |
|---|---|---|---|---|
| P131T_1.56% | 10200 | 130 | 655 | 15 |
| P131T _0.39% | 7080 | 76 | 634 | 9 |
| P131T _0.1% | 10000 | 84 | 670 | 10 |
| P174T_1.56% | 7240 | 147 | 603 | 18 |
| P174T_0.39% | 9100 | 73 | 627 | 9 |
| P174T _0.1% | 10800 | 126 | 598 | 15 |

**Table 9. Sequencing results of sample P131T prepared at three different dilution levels using the automation method according to the invention.**

| Expected frequency of the alternative allele P131T *LZTR1* - variant c.1197del | Detected frequency of the alternative allele | Depth of coverage for the alternative allele | Total sequencing depth at this position |
|---|---|---|---|
| 1.56% | 1.26% | 67 | 5336 |
| 0.39% | 0.44% | 16 | 3632 |
| 0.10% | 0.06% | 3 | 4649 |

**Table 10. Sequencing results of sample P174T prepared at three different dilution levels using the automation method according to the invention.**

| Expected frequency of the alternative allele P174T *SMARCBI* - Variant * 82C>T | Detected frequency of the alternative allele | Depth of coverage for the alternative allele | Total sequencing depth at this position |
|---|---|---|---|
| 1.56% | 1.30% | 80 | 6139 |
| 0.39% | 0.42% | 16 | 3819 |
| 0.10% | 0.05% | 3 | 5685 |

### Example 4

### Duplex sequencing method according to the invention using a hybridization panel targeting the TSC1/TSC2 genes loci

Similarly to Examples 2 and 3, the lowest frequencies of alternative alleles detectable using the procedure according to the invention, with a panel targeting two genes TSC1 and *TSC2,* associated with the molecular pathogenesis of tuberous sclerosis, were assessed. The procedure was carried out as described in Examples 2 and 3, maintaining an initial DNA concentration of 1600 ng. Table 11 presents the results obtained at the particular enzymatic stages, while data for the subsequent stages, including amplification and hybridization, are shown in Table 12. The sequencing results, presented in Tables 13 and 14, reveal minimal discrepancies between the expected and detected frequencies of alternative alleles. However, the reduction in allele frequency proceeded in accordance with the assumed dilution scheme (Fig. 1), and the obtained data confirm the automation method's effectiveness in detecting variants with frequencies as low as 0.06%, demonstrating its capability to identify alternative alleles at frequencies below 0.39%.

**Table 11. Summary of concentration values for the method according to the invention using a hybridization panel targeting the TSC1 and TSC2 gene loci.**

| **Sample ID** | USER (ng) | Loss 1 (%) | FPG (ng) | Loss 2 (%) | Mung (ng) | Loss 3 (%) | A-tail (ng) | Loss 4 (%) | Ligatio n (ng) | Loss 5 (%) |
|---|---|---|---|---|---|---|---|---|---|---|
| P59 0.39% | 1350 | 9 | 873 | 41 | 708 | 52 | 432 | 71 | 345 | 77 |
| P59 0.1% | 1452 | 4 | 949 | 37 | 745 | 51 | 384 | 75 | 338 | 78 |
| P1_0.39% | 1575 | 5 | 1000 | 33 | 670 | 55 | 483 | 68 | 368 | 75 |
| P1_0.1% | 1530 | 5 | 955 | 40 | 594 | 63 | 401 | 75 | 315 | 80 |
| **Mean** | **1477** | **8** | **944** | **41** | **679** | **58** | **425** | **73** | **342** | **79** |
| **Median** | **1491** | **7** | **952** | **41** | **689** | **57** | **417** | **73** | **342** | **79** |
| **Standard deviation** | **98.60** | **2.22** | **52.67** | **3.59** | **64.56** | **5.44** | **43.47** | **3.40** | **21.83** | **2.08** |

**Table 12. Comparison of amplification and hybridization efficiency for samples from two series with different concentrations processed with the automated procedure according to the invention, using a hybridization panel targeting the TSC1 and TSC2 gene loci.**

| **Sample ID** | After PCR1 (ng) | After PCR3 (ng) | Fragment length (bp) | Final library concentration (nM) |
|---|---|---|---|---|
| P59_0.39% | 5580 | 132 | 679 | 15 |
| P59_0.1% | 6480 | 146 | 659 | 17 |
| P1_0.39% | 9500 | 138 | 650 | 16 |
| P1_0.1% | 9720 | 152 | 636 | 18 |

**Table 13. Sequencing results of sample P1 prepared at two different dilution levels using the automation method according to the invention.**

| Expected frequency of the alternative allele P1 *TSC2* variant c.4630A>T | Detected frequency of the alternative allele | Depth of coverage for the alternative allele | Total sequencing depth at this position |
|---|---|---|---|
| 0.39% | 0.31% | 11 | 3584 |
| 0.10% | 0.09% | 3 | 3414 |

**Table 14. Sequencing results of sample P59 prepared at two different dilution levels using the automation method according to the invention.**

| Expected frequency of the alternative allele P59 *TSC1* variant c.2128C>T | Detected frequency of the alternative allele | Depth of coverage for the alternative allele | Total sequencing depth at this position |
|---|---|---|---|
| 0.39% | 0.24% | 7 | 2947 |
| 0.10% | 0.06% | 1 | 3308 |

### Example 5

### Reduced library preparation time for duplex sequencing using the method of the invention

The purpose of this analysis was to compare the time efficiency of duplex sequencing library preparation using both manual and automatic (method according to the invention) methods. The main objective was to assess the extent to which process automation accelerates the procedure and reduces the operator's workload. The enzymatic steps in both versions of the protocol include conducting reactions for a maximum of 8 samples simultaneously. In the case of the manual method, processing 8 samples presents an operational challenge and requires an experienced operator. This is due to the need for using specific reagent volumes outlined in the protocol, which necessitate the use of 1.5 mL tubes. This setup prevents the use of a multichannel pipette, limiting the number of samples that can be processed at the same time. As a result, the procedure time is prolonged, and the risk of uneven drying or over drying of magnetic beads during the purification steps increases. This may lead to material degradation, ultimately compromising the quality and quantity of the final libraries.

The method according to the invention allows for the simultaneous processing of all samples (up to 16), ensuring uniform pipetting. This not only shortens the overall procedure duration but also minimizes the risk of beads drying out and material loss during purification stages, ultimately leading to a higher quality of the final product.

In the example shown in Fig. 2, the total operator work time in the enzymatic part was reduced by 23% when using the method according to the invention compared to the manual method. The comparison of sample preparation times was divided into two stages: first, including enzymatic steps, and second comprising hybridization, which is more challenging in the manual version of the protocol. By using the automatic method, the user gains the ability to process up to 16 samples simultaneously, significantly increasing laboratory efficiency. This eliminates the need to repeat individual steps multiple times, as is required in the manual method, where restrictive reaction conditions-such as maintaining a constant high temperature during library purification after hybridization-limit the optimal number of samples that can be processed at once to only 4.

Fig. 3 demonstrates a comparison of the total work time required by a single operator between using the manual method versus the method according to the invention, across the steps of hybridization, purification, and amplification. This step of library preparation in the method according to the invention was shortened 5-fold, highlighting a significant advantage of automation.

The total time required to perform the process manually is 6790 minutes, equivalent to over 113 hours of work by one operator. By implementing automation, this time is reduced to 2460 minutes (approximately 16 hours), resulting in a 64% reduction. This time reductions leads to substantial resource savings and enhances laboratory throughput, enabling simultaneous analysis of a greater number of samples.

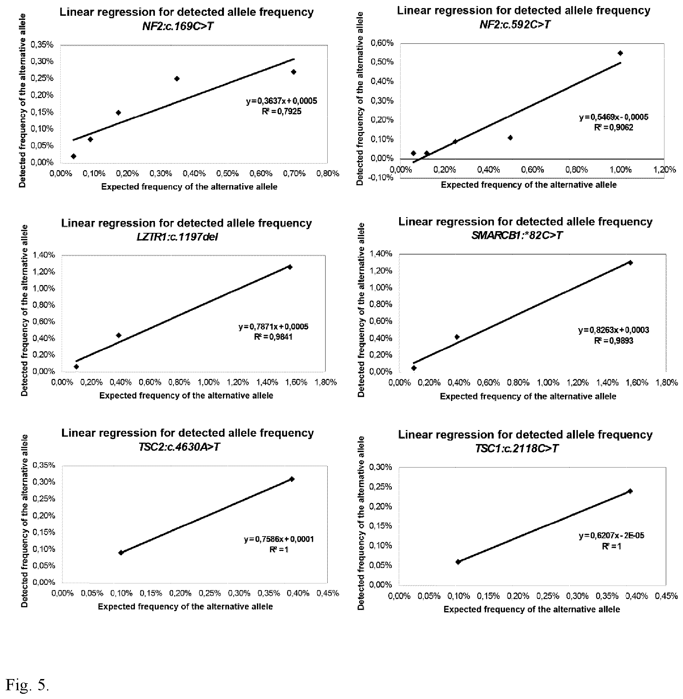

Furthermore, automation minimizes the need for repetitive tasks, which reduces the operator's workload and lowers the risk of errors associated with manual sample processing (Fig. 4). As a result, the entire process is streamlined, enhancing its efficiency (Fig. 5).

## Claims

1. A method for analyzing a biological material sample using a pipetting station for detection of mosaic genetic variants at a very low frequency in genes, involving the isolation of DNA from a peripheral blood sample, followed by the preparation of DNA libraries for duplex sequencing and further bioinformatic analysis, **characterized in that**:
a) DNA samples are prepared and subjected to mechanical DNA fragmentation using an ultrasonic device,
b) the resulting DNA fragments of approximately 500 base pairs are subjected to enzymatic reactions, adapter ligation, incubation, and purification using magnetic beads, wherein the enzymatic reactions include repairing the ends of fragmented DNA, ligation of the dedicated adapters, amplification using PCR reactions, and then
c) dedicated molecular probes targeting the human genome region are amplified and hybridized, and then
d) the quality and quantity of the obtained DNA libraries are verified, followed by sequencing, and the obtained data are analyzed using bioinformatic tools to generate a list of genetic variants along with their frequency in the studied gene(s) at the final stage of the analysis,
wherein steps b) and c) of the method are carried out using a Bravo NGS pipetting station from Agilent Technologies.

2. The method according to claim 1, **characterized in that** the hybridization in step c) is carried out using molecular probes covering coding regions, adjacent non-coding sequences flanking 50 nucleotides on both sides of exons, and regions of selected pathogenic variants deeply located in intronic sequences of the selected genes *TSC1, TSC2, NF2, LZTR1,* and *SMARCB1,* wherein the *TSC1* and *TSC2* genes are related to the molecular pathogenesis of tuberous sclerosis, and the *NF2, LZTR1,* and *SMARCBI* genes are related to the molecular pathogenesis of schwannomatosis.

3. The method according to any of claims 1 to 2, **characterized in that** it detects mosaicism variants at a low-level in the range of 1 to 5% and at a very low-level <1% in DNA isolated from peripheral blood leukocytes from patients suspected of selected neurocutaneous diseases.
